(19) Europäisches Patentamt
European Patent Office
Office européen des brevets

(11) Veröffentlichungsnummer: **0 562 261 A2**

(12) # EUROPÄISCHE PATENTANMELDUNG

(21) Anmeldenummer: **93102324.6**

(22) Anmeldetag: **15.02.93**

(51) Int. Cl.5: **C07D 233/68**, C07D 403/12, C07D 401/12, C07D 233/64, A61K 31/415

(30) Priorität: **27.02.92 DE 4206043**

(43) Veröffentlichungstag der Anmeldung: **29.09.93 Patentblatt 93/39**

(84) Benannte Vertragsstaaten: **AT BE CH DE DK ES FR GB GR IE IT LI LU MC NL PT SE**

(71) Anmelder: **BAYER AG**

**D-51368 Leverkusen(DE)**

(72) Erfinder: **Hanko, Rudolf, Dr.**
**Schillerstrasse 23**
**W-4000 Düsseldorf(DE)**
Erfinder: **Dressel, Jürgen, Ph.D.**
**Clausiusweg 9**
**W-5600 Wuppertal(DE)**
Erfinder: **Fey, Peter, Dr.**
**Am Eickhof 23**
**W-5600 Wuppertal(DE)**
Erfinder: **Hübsch, Walter, Dr.**
**Am Eckbusch 43/50**
**W-5600 Wuppertal(DE)**
Erfinder: **Krämer,Thomas, Dr.**
**In den Birken 92a**
**W-5600 Wuppertal(DE)**
Erfinder: **Müller, Ulrich E., Dr.**
**Claudiusweg 9**
**W-5600 Wuppertal(DE)**
Erfinder: **Müller-Gliemann, Matthias, Dr.**
**Claudiusweg 5**
**W-5600 Wuppertal(DE)**
Erfinder: **Beuck, Martin, Dr.**
**Trills 7**
**W-4006 Erkrath 2(DE)**
Erfinder: **Kazda, Stanislav, Prof.Dr.**
**Gellertweg 18**
**W-5600 Wuppertal(DE)**
Erfinder: **Hirth-Dietrich, Claudia Dr.**
**Claudiusweg 9**
**W-5600 Wuppertal(DE)**
Erfinder: **Knorr, Andreas, Dr.**
**Trillser Graben 10**
**W-4006 Erkrath 2(DE)**
Erfinder: **Stasch, Johannes-Peter, Dr.**
**Schneewittchenweg 37**
**W-5600 Wuppertal(DE)**
Erfinder: **Wohlfeil, Stefan, Dr.**
**Tucherweg 25**
**W-4010 Hilden(DE)**
Erfinder: **Yalkinoglu, Özkan, Dr.**
**Neuer Weg 21**
**W-5600 Wuppertal(DE)**

(54) **Sulfonybenzyl-substituierte Imidazole.**

(57) Sulfonylzbenzyl-substituierte Imidazole können hergestellt werden, indem man Imidazolylaldehyde zunächst mit Sulfonylbenzylverbindungen umsetzt und anschließend die Aldehydfunktion wie üblich oxidiert oder reduziert.

Die Sulfonylbenzyl-substituierten Imidazole können als Wirkstoffe in Arzneimitteln verwendet werden.

EP 0 562 261 A2

Die Erfindung betrifft Sulfonylbenzyl-substituierte Imidazole, ein Verfahren zu ihrer Herstellung sowie ihre Verwendung in Arzneimitteln, insbesondere als blutdrucksenkendes und anti-atherosklerotisches Mittel.

Es ist bekannt, daß Renin, ein proteolytisches Enzym, in vivo vom Angiotensinogen das Dekapeptid Angiotensin I abspaltet, welches wiederum in der Lunge, den Nieren oder anderen Geweben zu dem blutdrucksteigernden Oktapeptid Angiotensin II abgebaut wird. Die verschiedenen Effekte des Angiotensin II, wie beispielsweise Vasokonstriktion, $Na^+$-Retention in der Niere, Aldosteronfreisetzung in der Nebenniere und Tonuserhöhung des sympathischen Nervensystems wirken synergistisch im Sinne einer Blutdruckerhöhung.

Darüber hinaus besitzt Angiotensin II die Eigenschaft, das Wachstum und die Vermehrung von Zellen wie beispielsweise von Herzmuskelzellen und glatten Muskelzellen zu fördern, wobei diese bei verschiedenen Kranheitszuständen (z.B. Hypertonie, Atherosklerose und Herzinsuffizienz) vermehrt wachsen und proliferieren.

Ein möglicher Ansatz zum Eingriff in das Renin-Angiotensin-System (RAS) ist neben der Inhibierung der Reninaktivität die Hemmung der Aktivität des Angiotensin-Konversionsenzyms (ACE) sowie die Blockade von Angiotensin II-Rezeptoren.

In den Publikationen EP 324 377 A2, EP 403 158 A2 und EP 403 159 A2 werden Phenyl(alkyl)-imidazole-und Imidazolylalken-Säuren beschrieben.

Die vorliegende Erfindung betrifft Verbindungen der allgemeinen Formel (I)

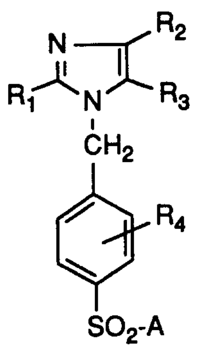

(I),

in welcher

| | |
|---|---|
| $R^1$ | für geradkettiges oder verzweigtes Alkyl oder Alkenyl mit jeweils bis zu 8 Kohlenstoffatomen steht, die gegebenenfalls durch Cycloalkyl mit 3 bis 6 Kohlenstoffatomen substituiert sind, oder für Cycloalkyl mit 3 bis 8 Kohlenstoffatomen steht, |
| $R^2$ | für Wasserstoff, Halogen oder für geradkettiges oder verzweigtes Perfluoralkyl mit bis zu 8 Kohlenstoffatomen steht, |
| $R^3$ | für geradkettiges oder verzweigtes Alkyl mit bis zu 6 Kohlenstoffatomen steht, das durch Hydroxy oder geradkettiges oder verzweigtes Alkoxy mit bis zu 4 Kohlenstoffatomen substituiert ist, oder für eine Gruppe der Formel $-CO-R^5$ oder $-CO-NR^6R^7$ steht, worin |
| $R^5$ | Wasserstoff, geradkettiges oder verzweigtes Alkoxy mit bis zu 8 Kohlenstoffatomen, Hydroxy, Benzyloxy oder Phenoxy bedeutet, |
| $R^6$ und $R^7$ | gleich oder verschieden sind und Wasserstoff, geradkettiges oder verzweigtes Alkyl mit bis zu 8 Kohlenstoffatomen oder Phenyl bedeuten, |
| $R^4$ | für Wasserstoff, Halogen oder geradkettiges oder verzweigtes Alkyl mit bis zu 8 Kohlenstoffatomen, oder<br>für geradkettiges oder verzweigtes Perfluoralkyl mit bis zu 6 Kohlenstoffatomen steht, oder<br>für eine Gruppe der Formel -OX steht, worin |
| X | Wasserstoff, Benzyl, eine Hydroxyschutzgruppe oder geradkettiges oder verzweigtes Alkyl mit bis zu 8 Kohlenstoffatomen bedeutet, |
| A | für einen über das Stickstoffatom gebundenen 3 bis 8-gliedrigen, gesättigten Heterocyclus mit bis zu 2 weiteren Heteroatomen aus der Reihe S, N oder O steht und der gegebenenfalls bis zu 2-fach gleich oder verschieden durch Perfluoralkyl mit bis zu 5 Kohlenstoffatomen oder durch einen Rest der Formel |

$-SO_3H$,

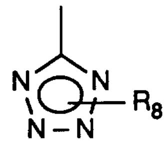,

$-CO-R^9$, $-CONR^{10}R^{11}$ oder

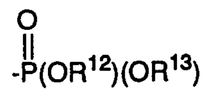

substituiert ist, worin

$R^8$ Wasserstoff, geradkettiges oder verzweigtes Alkyl mit bis zu 6 Kohlenstoffatomen oder Triphenylmethyl bedeutet

$R^9$ die oben angegebene Bedeutung von $R^5$ hat und mit dieser gleich oder verschieden ist

$R^{10}$ und $R^{11}$ die oben angegebene Bedeutung von $R^6$ und $R^7$ haben und mit diesen gleich oder verschieden sind,
und

$R^{12}$ und $R^{13}$ gleich oder verschieden sind und Wasserstoff, geradkettiges oder verzweigtes Alkyl mit bis zu 8 Kohlenstoffatomen oder Phenyl bedeuten,

und deren Salze.

Die erfindungsgemäßen Sulfonylbenzyl-substituierten Imidazole können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der Sulfonylbenzyl-substituierten Imidazole können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen, sein. Besonders bevorzugt sind z.B. Natrium-, Kalium-, Magnesium- oder Calciumsalze, sowie Ammoniumsalze, die abgeleitet sind von Ammoniak oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in stereoisomeren Formen, entweder als Enantiomere oder als Diastereomere, existieren. Die Erfindung betrifft sowohl die Enantiomeren oder Diastereomeren als auch deren jeweiligen Mischungen. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Ein über N gebundener, 3- bis 8-gliedriger gesättigter Heterocyclus, der außerdem als Heteroatome bis zu 2 Sauerstoff-, Schwefel- und/oder Stickstoffatome enthalten kann, steht im allgemeinen für Azetidinyl, Piperidyl, Morpholinyl, Piperazinyl oder Pyrrolidinyl. Bevorzugt sind 5- und 6-gliedrige Ringe mit einem Sauerstoff- und/oder bis zu 2 Stickstoffatomen, wie beispielsweise Piperidyl, Morpholinyl oder Pyrrolidinyl. Besonders bevorzugt sind Piperidyl und Pyrrolidinyl.

Bevorzugt sind Verbindungen der allgemeinen Formel (I),
in welcher

$R^1$ für geradkettiges oder verzweigtes Alkyl oder Alkenyl mit jeweils bis zu 6 Kohlenstoffatomen steht, die gegebenenfalls durch Cyclopropyl, Cyclobutyl, Cyclopentyl oder Cyclohexyl substituiert sind, oder für Cyclopropyl, Cyclobutyl, Cyclopentyl oder Cyclohexyl steht,

$R^2$ für Wasserstoff, Fluor, Chlor, Brom oder für geradkettiges oder verzweigtes Perfluoralkyl mit bis zu 6 Kohlenstoffatomen steht,

$R^3$ für geradkettiges oder verzweigtes Alkyl mit bis zu 4 Kohlenstoffatomen steht, das durch

Hydroxy, Methoxy oder Ethoxy substituiert ist, oder für eine Gruppe der Formel $-CO-R^5$ oder $-CO-NR^6R^7$ steht,
worin

$R^5$ Wasserstoff, geradkettiges oder verzweigtes Alkoxy mit bis zu 6 Kohlenstoffatomen, Hydroxy, Benzyloxy oder Phenoxy bedeutet,

$R^6$ und $R^7$ gleich oder verschieden sind und Wasserstoff oder geradkettiges oder verzweigtes Alkyl mit bis zu 6 Kohlenstoffatomen bedeuten,

$R^4$ für Wasserstoff, Fluor, Chlor, Brom, geradkettiges oder verzweigtes Alkyl mit bis zu 6 Kohlenstoffatomen, oder
für geradkettiges oder verzweigtes Perfluoralkyl mit bis zu 4 Kohlenstoffatomen steht, oder
für eine Gruppe der Formel -OX steht,
worin

X Wasserstoff, Benzyl, Acetyl oder geradkettiges oder verzweigtes Alkyl mit bis zu 6 Kohlenstoffatomen bedeutet,

A für über das Stickstoffatom gebundenes Azetidinyl, Piperidyl, Pyrrolidinyl oder Morpholinyl steht, die gegebenenfalls durch Trifluormethyl oder durch einen Rest der Formel
$-SO_3H$,

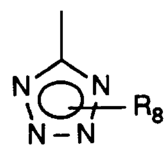
,

$-CO-R^9$, $-CONR^{10}R^{11}$ oder

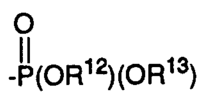

substituiert sind,
worin

$R^8$ Wasserstoff, geradkettiges oder verzweigtes Alkyl mit bis zu 4 Kohlenstoffatomen oder Triphenylmethyl bedeutet,

$R^9$ die oben angegebene Bedeutung von $R^5$ hat und mit diesem gleich oder verschieden ist,

$R^{10}$ und $R^{11}$ die oben angegebene Bedeutung von $R^6$ und $R^7$ haben und mit diesen gleich oder verschieden sind,
und

$R^{12}$ und $R^{13}$ gleich oder verschieden sind und Wasserstoff, geradkettiges oder verzweigtes Alkyl mit bis zu 6 Kohlenstoffatomen oder Phenyl bedeuten,

und deren Salze.

Besonders bevorzugt sind Verbindungen der allgemeinen Formel (I),
in welcher

$R^1$ für geradkettiges oder verzweigtes Alkyl oder Alkenyl mit jeweils bis zu 4 Kohlenstoffatomen oder Cyclopropyl steht,

$R^2$ für Wasserstoff, Fluor, Chlor oder für geradkettiges oder verzweigtes Perfluoralkyl mit bis zu 4 Kohlenstoffatomen steht,

$R^3$ für die $-CH_2OH$-Gruppe steht, oder
für eine Gruppe der Formel $-CO-R^5$ oder $-CO-NR^6R^7$ steht,
worin

$R^5$ Wasserstoff, geradkettiges oder verzweigtes Alkoxy mit bis zu 4 Kohlenstoffatomen, Hydroxy, Benzyloxy oder Phenoxy bedeutet,

$R^6$ und $R^7$ gleich oder verschieden sind und Wasserstoff oder geradkettiges oder verzweigtes Alkyl mit bis zu 4 Kohlenstoffatomen bedeuten,

$R^4$ für Wasserstoff, Fluor, Chlor, geradkettiges oder verzweigtes Alkyl mit bis zu 4 Kohlenstoffatomen, oder
für geradkettiges oder verzweigtes Perfluoralkyl mit bis zu 3 Kohlenstoffatomen steht,
oder
für eine Gruppe der Formel -OX steht,
worin

X Wasserstoff, Benzyl, Acetyl oder geradkettiges oder verzweigtes Alkyl mit bis zu 6 Kohlenstoffatomen bedeutet,

A für über das Stickstoffatom gebundenes Piperidyl, Pyrrolidinyl steht, die gegebenenfalls durch Trifluormethyl oder durch einen Rest der Formel

$-SO_3H$,

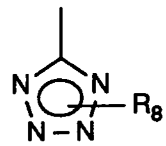 ,

$-CO-R^9$, $-CONR^{10}R^{11}$ oder

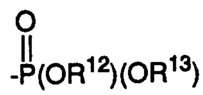

substituiert sind,
worin

$R^8$ Wasserstoff, Methyl, Ethyl oder Triphenyl bedeutet,

$R^9$ die oben angegebene Bedeutung von $R^5$ hat und mit diesem gleich oder verschieden ist,

$R^{10}$ und $R^{11}$ die oben angegebene Bedeutung von $R^6$ und $R^7$ haben und mit diesen gleich oder verschieden sind,
und

$R^{12}$ und $R^{13}$ gleich oder verschieden sind und Wasserstoff, geradkettiges oder verzweigtes Alkyl mit bis zu 4 Kohlenstoffatomen oder Phenyl bedeuten,

und deren Salze.

Außerdem wurde ein Verfahren zur Herstellung der erfindungsgemäßen Verbindungen der allgemeinen Formel (I) gefunden, dadurch gekennzeichnet, daß man Aldehyde der allgemeinen Formel (II)

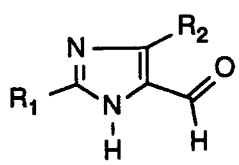 (II),

in welcher
$R^1$ und $R^2$ die oben angegebene Bedeutung haben,
zunächst mit Verbindungen der allgemeinen Formel (III)

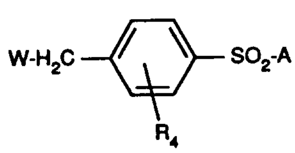 (III),

in welcher
$R^4$ und A die oben angegebene Bedeutung haben
und
W für Halogen, vorzugsweise für Brom steht,
in inerten Lösemitteln, gegebenenfalls in Anwesenheit einer Base umsetzt,
und anschließend die Aldehydfunktion, gegebenenfalls nach üblichen Methoden oxidiert oder reduziert, im Fall der Ester, die Säuren nach üblicher Methode auch nachträglich verestert bzw. im Fall der Säuren, die Ester verseift,
im Fall der Amide, eine Amidierung, gegebenenfalls über eine aktivierte Carbonsäurestufe, in Anwesenheit einer Base und/oder eines Dehydratisierungsstoffes und im Fall, daß $R^8 \neq H$ eine Alkylierung, anschließt.

Das erfindungsgemäße Verfahren kann durch folgendes Formelschema beispielhaft erläutert werden:

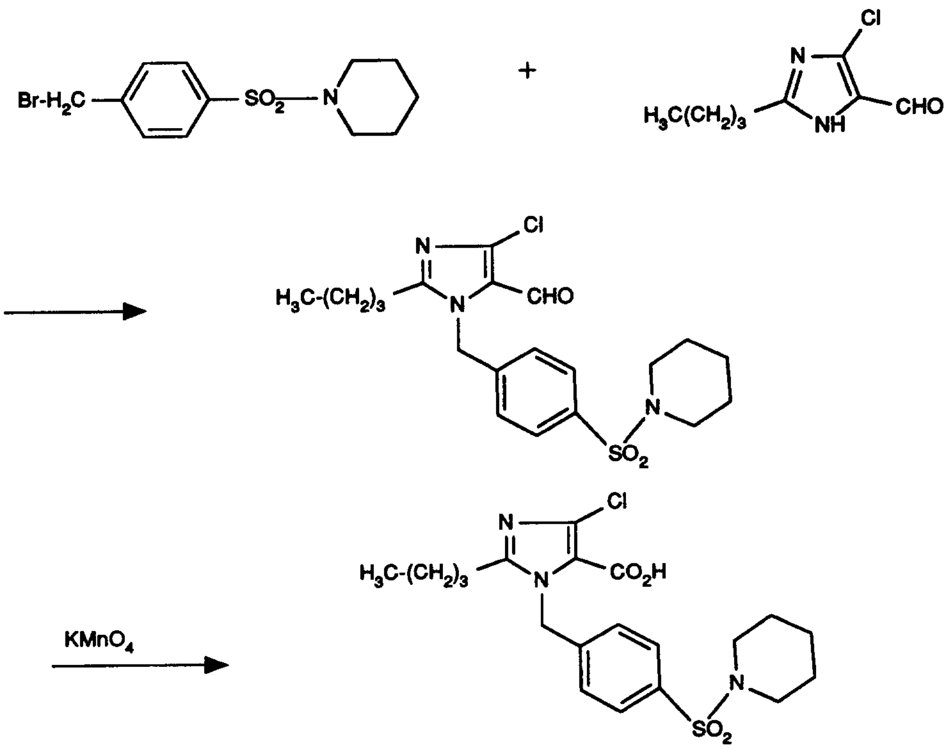


Als Lösemittel für die einzelnen Schritte des Verfahrens eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfrakionen, oder Halogenkohlenwasserstoffe wie Dichlormethan, Trichlormethan, Tetrachlormethan, Dichlorethylen, Trichlorethylen oder Chlorbenzol, oder Essigester, oder Alkohole wie Methanol, Ethanol oder tert.Butanol, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind für die verschiedenen Schritte Tetrahydrofuran, Methylenchlorid, Toluol und Dimethylformamid.

Als Basen können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali- oder Erdalkalialkoholate oder -amide wie Natrium- oder Kaliummethanolat, Natrium- oder Kaliumethanolat oder Kalium-tert.butylat oder Lithiumdiisopropylamid (LDA), oder organische Amine (Trialkyl($C_1$-$C_6$)amine) wie Triethylamin oder N,N-Diisopropylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich, als Basen Alkalimetalle, wie Natrium oder deren Hydride wie Natriumhydrid, einzusetzen. Bevorzugt ist Natriumhydrid.

Im allgemeinen setzt man die Base in einer Menge von 0,05 mol bis 10 mol, bevorzugt von 1 mol bis 2 mol bezogen auf 1 mol der Verbindung der Formel (III) ein.

Das erfindungsgemäße Verfahren wird im allgemeinen in einem Temperaturbereich von -20 °C bis +100 °C, bevorzugt von 0 °C bis +80 °C durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Oxidation erfolgt im allgemeinen in einem der oben aufgeführten Lösemitteln, vorzugsweise mit tert.Butanol mit Oxidationsmitteln wie beispielsweise Kaliumpermanganat, Chromylchlorid, Cerammoniumnitrat, Silberoxid, Selendioxid oder ein Chrom(VI)oxid in Verbindung mit Essigsäureanhydrid. Bevorzugt ist Kaliumpermanganat.

Die Oxidation kann bei Normaldruck oder erhöhtem oder erniedrigtem Druck (beispielsweise von 0,5 bis 5 bar), vorzugsweise bei Normaldruck durchgeführt werden. Sie erfolgt im einem Temperaturbereich von 0 °C bis +40 °C, vorzugsweise bei Raumtemperatur.

Die Reduktion von Alkoxycarbonylverbindungen oder Aldehyden zu den entsprechenden Alkoholen erfolgt im allgemeinen mit Hydriden, wie Lithiumaluminiumhydrid oder Natriumborhydrid, bevorzugt mit Natriumborhydrid in inerten Lösemitteln wie Ethern, Kohlenwasserstoffen oder Alkoholen oder deren Gemischen, bevorzugt in Ethern wie beispielsweise Diethylether, Tetrahydrofuran oder Dioxan, oder Alkoholen wie Ethanol, im Fall der Aldehyde bevorzugt mit Natriumborhydrid in Tetrahydrofuran, in einem Temperaturbereich von 0 °C bis +150 °C, bevorzugt von +20 °C bis +80 °C, bei Normaldruck.

Als Lösemittel für die Alkylierung eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Dichlormethan.

Die Alkylierung wird in den oben aufgeführten Lösemitteln bei Temperaturen von 0 °C bis +150 °C, vorzugsweise bei Raumtemperaturen bis +100 °C, bei Normaldruck durchgeführt.

Die Amidierung erfolgt im allgemeinen in inerten Lösemitteln in Anwesenheit einer Base und eines Dehydratisierungsmittels.

Als Lösemittel eignen sich hierbei inerte organische Lösermittel, die sich unter den Reaktionsbedingungen nicht verändern. Hierzu gehören Halogenkohlenwasserstoffe wie Dichlormethan, Trichlormethan, Tetrachlormethan, 1,2-Dichlorethan, Trichlorethan, Tetrachlorethan, 1,2-Dichlorethan oder Trichlorethylen, Kohlenwassserstoffe wie Benzol, Xylol, Toluol, Hexan, Cyclohexan, oder Erdölfraktionen, Nitromethan, Dimethylformamid, Acetonitril oder Hexamethylphosphorsäuretriamid. Ebenso ist es möglich, Gemische der Lösemittel einzusetzen. Besonders bevorzugt ist Dichlormethan.

Als Basen für die Amidierung eignen sich die üblichen basischen Verbindungen. Hierzu gehören vorzugsweise Alkali- und Erdalkalihydroxide wie Lithiumhydroxid, Natriumhydroxid, Kaliumhydroxid oder Bariumhydroxid, Alkalihydride wie Natriumhydrid, Alkali- oder Erdalkalicarbonate wie Natriumcarbonat, Kaliumcarbonat, oder Alkalialkoholate wie beispielsweise Natriummethanolat oder -ethanolat, Kaliummethanolat oder -ethanolat oder Kalium-tert.butylat, oder organische Amine wie Benzyltrimethylammoniumhydroxid, Tetrabutylammoniumhydroxid, Pyridin, Triethylamin oder N-Methylpiperidin.

Die Amidierung wird im allgemeinen in einem Temperaturbereich von 0 °C bis 150 °C, bevorzugt bei 25 °C bis 40 °C, durchgeführt.

Die Amidierung wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Bei der Durchführung der Amidierung wird die Base im allgemeinen in einer Menge von 1 bis 3 Mol, bevorzugt von 1 bis 1,5 Mol, bezogen auf 1 Mol der entsprechenden Carbonsäure, eingesetzt.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphorsäureanhydrid oder Isobutylchlorolormat oder Benzotriazolyloxy-tris-(dimethylamino)phosphonium-hexafluorophosphat oder Phosphonsäurediphenylesteramid oder Methansulfonsäurechlorid oder Thionylchlorid, gegebenenfalls in Anwesenheit von Basen wie Triethylamin oder N-Ethylmorpholin oder N-Methylpiperidin oder Dicyclohexylcarbodiimid und N-Hydroxysuccinimid [vgl. J.C. Sheehan, S.L. Ledis, J. Am. Chem. Soc. 95, 875 (1973); F.E. Freeman et al., J. Biol. Chem. 225, 507 (1982) und N.B. Benoton, K. Kluroda, Int. Pept. Prot. Res. 13, 403 (1979), 17, 187 (1981)].

Die Veresterung erfolgt nach üblichen Methoden, indem man die entsprechende Carbonsäure ($R^5$ = OH), gegebenenfalls auch über eine aktivierte Stufe, mit den entsprechenden Alkoholen, in einem Temperaturbereich von -20 °C bis +120 °C, vorzugsweise bei 20 °C, und Normaldruck umsetzt.

Die Verseifung der Carbonsäureester erfolgt nach üblichen Methoden, indem man die Ester in inerten Lösemitteln mit üblichen Basen behandelt.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofüran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0 °C bis +100 °C, bevorzugt von +20 °C bis +80 °C, durchgeführt.

Im allgemeinen wird die Verseifung bei Normaldruck durchgeführt Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 Mol, bevorzugt von 1 bis 1,5 Mol bezogen auf 1 Mol des Esters, eingesetzt. Besonders bevorzugt verwendet man molare Mengen der Reaktanden.

Die Verbindungen der allgemeinen Formel (III) sind neu und können hergestellt werden, indem man substituierte Benzylsulfonsäurechloride der allgemeinen Formel (IV)

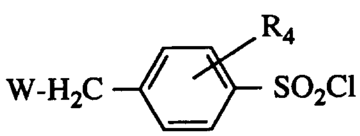

(IV),

in welcher

$R^4$ und W die oben angegebene Bedeutung haben,

mit Verbindungen der allgemeinen Formel (V)

H-A (V),

in welcher

A die oben angegebene Bedeutung hat,

in einem der oben angegebenen Lösemittel und Basen, vorzugsweise in Dichlormethan mit Triethylamin umsetzt.

Im allgemeinen wird die Umsetzung bei Normaldruck durchgeführt. Es ist aber auch möglich, bei Unterdruck oder bei Überdruck zu arbeiten (z.B. von 0,5 bis 5 bar).

Bei der Durchführung der Umsetzung wird die Base im allgemeinen in einer Menge von 1 bis 3 mol, bevorzugt von I bis 1,5 mol bezogen auf 1 mol der Verbindungen der allgemeinen Formel (IV), eingesetzt. Besonders bevorzugt verwendet man molare Mengen der Reaktanden.

Die Umsetzung erfolgt im allgemeinen in einem Temperaturbereich von -40 °C bis +40 °C, vorzugsweise von -30 °C bis 0 °C und unter Normaldruck.

Die Verbindungen der allgemeinen Formeln (IV) und (V) sind bekannt oder können nach üblicher Methode hergestellt werden.

Die Verbindungen der allgemeinen Formel (II) sind bekannt oder können nach üblicher Methode hergestellt werden [vgl. z.B. Beilstein 9, 511].

Die erfindungsgemäßen Verbindungen der allgemeinen Formel (I) zeigen ein nicht vorhersehbares, wertvolles pharmakologisches Wirkspektrum.

Die erfindungsgemäßen Verbindungen besitzen eine spezifische A II-antagonistische Wirkung, da sie die Bindung von Angiotensin II an die A II-Rezeptoren hemmen. Sie unterdrücken die vasokonstriktorischen und Aldosteronsekretionsstimulierenden Effekte des Angiotensin II. Darüberhinaus inhibieren sie die Proliferation von glatten Muskelzellen.

Sie können deshalb in Arzneimitteln zur Behandlung der arteriellen Hypertonie und Atherosklerose eingesetzt werden. Darüberhinaus können sie zur Behandlung von coronaren Herzerkrankungen, Herzinsuffizienz, Störungen der Hirnleistung, ischämischen Gehirnerkrankungen, peripherer Durchblutungsstörungen,

Funktionsstörungen der Niere und Nebenniere, bronchospastischen und vaskulär bedingten Erkrankungen der Atemwege, Natriumretention und Ödemen eingesetzt werden.

Darüberhinaus besitzen die Substanzen natriuretische und diuretische Wirkung. Diese Wirkung äußert sich in einer Odemausschwemmung bei pathologischer Flüssigkeitsvermehrung kardialen und nicht kardialen Ursprungs.

Untersuchung auf die Hemmung der mit Agonisten induzierten Kontraktion

Kaninchen beiderlei Geschlechts werden durch Nackenschlag betäubt und entblutet, oder fallweise mit Nembutal (ca. 60 - 80 mg/kg i.v.) narkotisiert und durch Öffnung des Thorax getötet. Die Thoraxaorta wird entnommen, von anhaftendem Bindegewebe befreit, in 1,5 mm breite Ringsegmente geteilt und einzeln unter einer Anfangsbelastung von ca. 3,5 g in 10 ml Organbäder mit auf 37°C temperierter, Carbogenbegaster Krebs-Henseleit-Nährlösung folgender Zusammensetzung: 119 mmol/l NaCl; 2,5 mmol/l $CaCl_2$ x 2 $H_2O$; 1,2 mmol/l $KH_2PO_4$; 10 mmol/l Glucose; 4,8 mmol/l KCl; 1,4 mmol/l $MgSO_4$ x 7 $H_2O$ und 25 mmol/l $NaHCO_3$ verbracht.

Die Kontraktionen werden isometrisch durch Statham UC2-Zellen über Brückenverstärker (ifd Mülheim bzw. DSM Aalen) erfaßt und mittels A/D-Wandler (System 570, Keithley München) digitalisiert sowie ausgewertet. Die Durchführung von Agonistdosiswirkungskurven (DWK) erfolgt stündlich. Mit jeder DWK werden 3 bzw. 4 Einzelkonzentrationen im 4 min-Abstand in die Bäder appliziert. Nach Ende der DWK und darauffolgender Auswaschzyklen (16 mal jeweils ca. 5 sec/min mit der o.a. Nährlösung) schließt sich eine 28-minütige Ruhe- bzw. Inkubationsphase an, innerhalb derer die Kontraktionen in der Regel wieder den Ausgangswert erreichen.

Die Höhe der im Normalfall 3. DWK wird als Bezugsgröße für die Bewertung der in weiteren Durchgängen zu untersuchenden Testsubstanz verwendet, die bei den nachfolgenden DWK's in jeweils steigender Dosierung mit Beginn der Inkubationszeit in die Bäder appliziert wird. Jeder Aortenring wird dabei ganztägig mit immer dem gleichen Agonisten stimuliert.

Agonisten und ihre Standardkonzentrationen

Applikationsvolumen pro Einzelgabe = 100 µl:

| | | |
|---|---|---|
| KCl | 22,7;32,7;42,7;52,7 | mmol/l |
| 1Noradrenalin | $3x10^{-9}$;$3x10^{-8}$;$3x10^{-7}$;$3x10^{-6}$ | g/ml |
| Serotonin | $10^{-8}$;$10^{-7}$;$10^{-6}$;$10^{-5}$ | g/ml |
| B-HT 920 | $10^{-7}$;$10^{-6}$;$10^{-5}$ | g/ml |
| Methoxamin | $10^{-7}$;$10^{-6}$;$10^{-5}$ | g/ml |
| Angiotensin II | $3x10^{-9}$;$10^{-8}$;$3x10^{-8}$;$10^{-7}$ | g/ml |

Für die Berechnung der $IC_{50}$ (Konzentration bei der die zu untersuchende Substanz eine 50%ige Hemmung verursacht) wird der Effekt jeweils an der 3. = submaximalen Agonistkonzentration zugrundegelegt.

Die erfindungsgemäßen Verbindungen hemmen die durch Angiotensin II induzierte Kontraktion der isolierten Kaninchenaorta dosenabhängig. Die durch Kalium-Depolarisation oder andere Agonisten induzierte Kontraktion wurde nicht oder in hohen Konzentrationen nur schwach gehemmt.

Tabelle A:

Hemmung der Gefäßkontraktion an isolierten Aortenringen von Kaninchen in vitro

$IC_{50}$ [nM] gegen Kontraktionen, induziert durch:

| Bsp.Nr.: | AII |
|---|---|
| 15 | 202 |
| 16 | 309 |
| 19 | 44 |
| 25 | 5 |
| 21 | 250 |

Blutdruckmessungen an der Angiotensin II-infundierten Ratte

Männliche Wistar Ratten (Moellegaard, Kopenhagen, Dänemark) mit einem Körpergewicht von 300 - 350 g, werden mit Thiopental (100 mg/kg i.p.) anästhesiert. Nach Tracheotomie wird in die Femoralarterie ein Katheter zur Blutdruckmessung und in die Femoralvenen ein Katheter für die Angiotensin II-Infusion und ein Katheter für die Substanzverabreichung eingeführt. Nach Gabe des Ganglienblockers Pentolinium (5 mg/kg i.v.) wird die Angiotensin II-Infusion (0,3 µg/kg/min) gestartet. Sobald die Blutdruckwerte ein stabiles Plateau erreicht haben, werden die Testsubstanzen entweder intravenös oder als Suspension bzw. Lösung in 0,5% Tylose oral verabreicht. Die Blutdruckveränderungen unter Substanzeinfluß sind als Mittelwerte ± SEM in der Tabelle angegeben.

Bestimmung der antihypertensiven Wirksamkeit bei wachen hypertensiven Ratten

Die orale antihypertensive Wirksamkeit der erfindungsgemäßen Verbindungen wurde an wachen Ratten mit chirurgisch induzierter unilateraler Nierenarterienstenose geprüft. Dazu wurde die rechte Nierenarterie mit einem Silberclip von 0,18 mm lichter Weite eingeengt. Bei dieser Hypertonieform ist die Plasmareninaktivität in den ersten sechs Wochen nach dem Eingriff erhöht.
Der arterielle Blutdruck diese Tiere wurde in definierten Zeitabständen nach Substanzgabe mit der "Schwanzmanschette" unblutig gemessen. Die zu prüfenden Substanzen wurden aufgeschwemmt in einer Tylosesuspension intragastral ("oral") per Schlundsonde in verschiedenen Dosen appliziert. Die erfindungsgemäßen Verbindungen senken den arteriellen Blutdruck der Hochdruckratten in klinisch relevanter Dosierung.

Außerdem hemmen die erfindungsgemäßen Verbindungen die spezifische Bindung von radioaktivem Angiotensin II konzentrationsabhängig.

Interaktion der erfindungsgemäßen Verbindungen mit dem Angiotensin II-Rezeptor an Membranfraktionen der Nebennierenrinde (Rind)

Nebennierenrinden vom Rind (NNR), die frisch entnommen und sorgfältig von Mark von Kapsel befreit sind, werden in Sucrose-Lösung (0,32 M) mit Hilfe eines Ultra-Turrax (Janke & Kunkel, Staufen i.B.) zu grobem Membran-Homogenat zerKleinert und in zwei Zentrifugationsschritten zu Membranfraktionen partiell aufgereinigt.
Die Untersuchungen zur Rezeptor-Bindung werden an partiell gereinigten Membranfraktionen boviner NNR mit radioaktivem Angiotensin II in einem Assay-Volumen von 0,25 ml durchgeführt, das im einzelnen die partiell gereinigten Membranen (50 - 80 µg), $^{3}H$-Angiotensin II (3-5 nM), Test-Pufferlösung (50 mM Tris, pH 7,2), 5 mM $MgCl_2$ sowie die zu untersuchenden Substanzen enthält. Nach einer Inkubationszeit von 60 min bei Raumtemperatur wird die nicht gebundene Radioaktivität der Proben mittels angefeuchteter Glasfaserfilter (Whatman GF/C) separiert und die gebundene Radioaktivität nach Waschung des Proteins mit eiskalter Pufferlösung (50 mM Tris/HCl, pH 7,4, 5% PEG 6000) in einem Szintillationscocktail spektrophotometrisch gemessen. Die Analyse der Rohdaten erfolgte mit Computer-Programmen zu $K_i$- bzw. $IC_{50}$-Werten ($K_i$: für die verwendete Radioaktivität korrigierte $IC_{50}$-Werte; $IC_{50}$-Werte: Konzentration bei der die zu untersuchende Substanz eine 50%ige Hemmung der spezifischen Bindung des Radioliganden bewirkt).
Bsp. Nr. 15 $K_i$ = 400 nM
Bsp. Nr. 18 $K_i$ = 200 nM

Untersuchung zur Inhibition der Proliferation glatter Muskelzellen durch die erfindungsgemäßen Verbindungen

Zur Feststellung der antiproliferativen Wirkung der Verbindungen werden glatte Muskelzellen verwendet, die aus den Aorten von Ratten oder Schweinen durch die Media-Explantat-Technik gewonnen werden [R. Ross, J. Cell. Biol. 50, 172, 1971]. Die Zellen werden in geeigneten Kulturschalen, in der Regel 24-Loch-Platten, ausgesät und für 2 - 3 Tage in Medium 199 mit 7,5% FCS und 7,5% NCS, 2 mM L-Glutamin und 15 mM HEPES, pH 7,4 in 5% $CO_2$ bei 37 °C Kultiviert Danach werden die Zellen durch Serumentzug für 2 - 3 Tage synchronisiert und sodann mit AII, Serum oder anderen Faktoren zum Wachstum angeregt. Gleichzeitig werden Testverbindungen zugesetzt. Nach 16 - 20 Stunden wird 1 µCi $^3H$-Thymidin zugefügt und nach weiteren 4 Stunden der Einbau dieser Substanz in die TCA-präzipitierbare DNA der Zellen bestimmt.

| Bsp. Nr. | % Hemmung bei $10^{-6}$ M |
|---|---|
| 11 | 20 |
| 12 | 70 |

Prüfung auf natriuretische Wirkung

Nüchterne Wistar-Ratten werden mit Prüfsubstanz (suspendiert in Tylose-Lösung) oral behandelt. Anschließend wird in Diurese-Käfigen die Harnausscheidung über 6 Std. gesammelt. Die Konzentration von Natrium und Kalium im Harn wird flammenphotometrisch bestimmt.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Losungen, unter Verwendung inerter, nichttoxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Losemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

| Laufmittelgemische: | |
|---|---|
| a = Petrolether/Essigester | 1:1 |
| b = $CH_2Cl_2$/Essigester | 5:1 |
| c = $CH_2Cl_2/CH_3OH$ | 3:1 |
| d = $CH_2Cl_2/CH_3OH$ | 10:1 |

Ausgangsverbindungen

Beispiel I

4-(Brommethyl)benzol-sulfochlorid

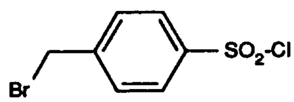

38,1 g (0,2 mol) 4-Methylbenzolsulfonylchlorid werden in 300 ml Tetrachlorkohlenstoff gelöst und mit 35,6 g (0,2 mol) N-Bromsuccinimid versetzt und nach Zugabe von 0,2 g (1,2 mmol) Azobisisobutyronitril (ABU) für 4 h unter Rückfluß erhitzt. Nach dem Abkühlen werden die Feststoffe abfiltriert und das Filtrat vom Lösemittel befreit. Flash-Chromatographie (Petrolether / Toluol 4:1, 50 µm Korngröße) und anschließende Umkristallisation aus 100 ml Cyclohexan ergibt 24,0 g (45% der Theorie) der Titelverbindung.
$R_f$ = 0,75 (Toluol)

Beispiel II

4-(Brommethyl)-3-chlorbenzolsulfochlorid

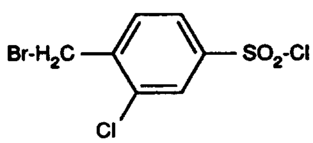

45,9 g (0,2 mol) 3-Chlor-4-methylbenzolsulfonsäure Natriumsalz werden mit 83,3 g (0,4 mol) Phosphorpentachlorid gemischt und 30 min bei 140°C Ölbadtemperatur erhitzt. In der Hitze wird mit 500 ml Toluol versetzt, die entstandene Lösung bis zum Sieden erhitzt und nach dem Abkühlen auf Eis gegeben. Die organische Phase wird abgetrennt und mit Wasser gewaschen (2 x 200 ml). Nach dem Trocknen über $MgSO_4$ wird filtriert und alles Flüchtige im Vakuum abgezogen. Der erhaltene Rückstand wird durch Flashchromatographie (Petrolether / Toluol 4:1, 50 µ Korngröße) gereinigt. Man erhält 24,9 g eines Produkts das sofort weiter umgesetzt wird:
Man nimmt in 200 ml Tetrachlorkohlenstoff auf und erhitzt nach Zugabe von 19,6 g (0,11 mol) N-Bromsuccinimid und 0,1 g (0,6 mmol) ABN für 6 h unter Rückfluß. Nach dem Abkühlen werden die Feststoffe abfiltriert und das Filtrat vom Lösemittel befreit. Flashchromatographie (Petrolether / Toluol 4:1, 50 µ Korngröße) ergibt 21,2 g (35%) der Titelverbindung.
$R_f$ = 0,32 (Petrolether / Dichlormethan 4:1)

Beispiel III

4-(Brommethyl)-benzolsulfonyl-N-pyrrolidinid

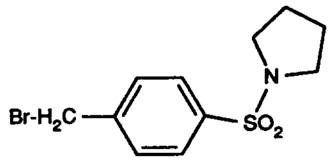

5,3 g (0,02 mol) der Verbindung aus Beispiel I werden in 200 ml Dichlormethan und 4,0 g (0,04 mol) Triethylamin gelöst und nach Zugabe von 1,4 g (0,02 mol) Pyrrolidin in 50 ml Dichlormethan bei 0 °C für 1 h bei 0 °C nachgerührt. Man extrahiert mit 2 N HCl (2 x 100 ml), $H_2O$ (2 x 100 ml), trocknet über $MgSO_4$, filtriert und verdampft alle flüchtigen Anteile im Vakuum.
Ausbeute: 5,4g (89% der Theorie)
$R_f$ = 0,09 (Toluol)

Beispiel IV

4-(Brommethyl)benzolsulfonyl-N-piperidinid

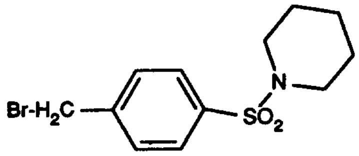

In Analogie zur Vorschrift des Beispiels III erhält man aus 1,1 g (4 mmol) der Verbindung aus Beispiel I und 0,34 g (4 mmol) Piperidin 1,0 g (81% der Theorie) der Titelverbindung.
$R_f$ = 0,14 (Toluol)

Beispiel V

(S)-4-(Brommethyl)-benzolsulfonyl-N-2-(tert.butoxycarbonyl)pyrrolidinid

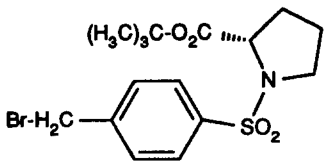

In Analogie zur Vorschrift des Beispiels III erhält man aus 7,25 g (27 mmol) der Verbindung aus Beispiel I und 4,6 g (27 mmol) S-Prolin-tert.butylester 9,1 g (84% der Theorie) der Titelverbindung.
$R_f$ = 0,66 (Petrolether / Essigester 7:3)

Beispiel VI

rac-4-(Brommethyl)-benzolsulfonyl-N-2-(tert.butoxycarbonyl)piperidinid

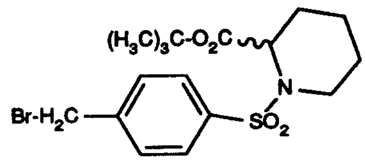

In Analogie zur Vorschrift des Beispiels III erhält man aus 8,0 g (30 mmol) der Verbindung aus Beispiel I und 5,5 g (30 mmol) rac-Pipecolinsäure-tert.butylester7,4 g (59% der Theorie) der Titelverbindung.
$R_f$ = 0,53 (Petrolether / Essigester 5:1)

Beispiel VII

(S)-4-(Brommethyl)-3-chlorbenzolsulfonyl-N-2-(tert.butoxycarbonyl)pyrrolidinid

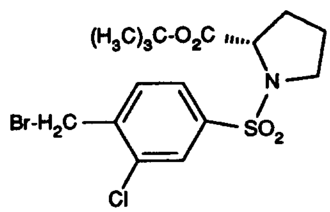

In Analogie zur Vorschrift des Beispiels III erhält man aus 10,0 g (33 mmol) der Verbindung aus Beispiel II und 5,7 g (33 mmol) S-Prolin-tert.butylester 13,9 g (96% der Theorie) der Titelverbindung.
$R_f$ = 0,55 (Petrolether / Essigester 7:3)

Beispiel VIII

rac-4-(Brommethyl)-3-chlorbenzolsulfonyl-N-2-(tert.butoxycarbonyl)piperidinid

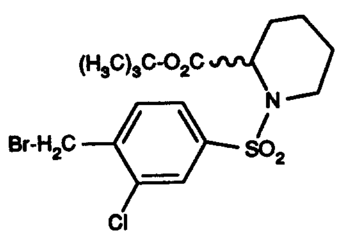

In Analogie zur Vorschrift des Beispiels III erhält man aus 10,0 g (33 mmol) der Verbindung aus Beispiel II und 6,1 g (33 mmol) rac-Pipecolinsäure-tert.butylester 14,6 g (98% der Theorie) der Titelverbindung.
$R_f$ = 0,6 (Petrolether / Essigester 7:3)

Beispiel IX

N-Trifluoracetyl-L-prolinamid

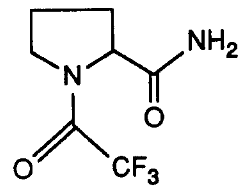

30 g (0,142 mol) Trifluoracetylprolin werden unter Schutzgas in 150 ml DMF vorgelegt. Bei -20 °C gibt man 142,6 ml (0,1704 mol) 38 %iges PPA in Essigester zu. Es wird bis zur Sättigung Ammoniak eingeleitet, wobei nach 30 min ein weißer Niederschlag ausfällt. Unter schwachem Ammoniakstrom wird der Ansatz aufgetaut. Dann gibt man die gesamte Reaktionsmischung in 600 ml $H_2O$ und säuert mit konzentrierter Essigsäure bis pH 4 an. Es wird 4 x mit 200 ml Methylenchlorid und 3 x mit 200 ml Ether ausgeschüttelt. Die vereinigten organischen Phasen werden mit Magnesiumsulfat getrocknet und das Lösungsmittel abgezogen. Die Rückstände chromatographiert man zusammen über Kieselgel 60 F254, Methylenchlorid/Methanol (10:1). Die das Produkt enthaltenden Fraktionen werden am Rotationsverdampfer vom Lösungsmittel befreit.
Man erhält 17,12 g der Titelverbindung (57 % der Theorie);
$R_f$: 0,345 (Touluen/Essigester/$CH_3COOH$) 20:20:1.

Beispiel X

2-Cyano-N-trifluoracetyl-pyrrolidin

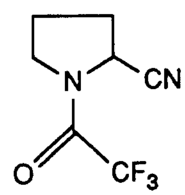

40 g (0,19 mol) des Produkts aus Beispiel IX und 45 g = 46 ml (0,57 mol) Pyridin legt man unter Schutzgas in 300 ml THF vor. Bei 0 °C tropft man 48 g = 32,25 ml (0,228 mol) Trifluoressigsäureanhydrid zu. Die Reaktionsmischung wird 30 min bei 0 °C und 90 min bei Raumtemperatur nachgerührt. Der Ansatz wird dann in 1 l 1N Salzsäure gegeben und 3 x mit 200 ml Methylenchlorid ausgeschüttelt. Die vereinigten organischen Phasen werden mit 200 ml gesättigter NaCl-Lösung ausgeschüttelt und über Magnesiumsulfat getrocknet. Das Lösungsmittel wird abgezogen und der Rückstand wird an Kieselgel 60 F254 chromatographiert. Petrolether Essigester/Essigsäure (1600:200:5). Die das Produkt enthaltenden Fraktionen engt man ein. Man erhält 32,4 g der Titelverbindung (88,8 % der Theorie).
$R_f$: 0,57 (Petroleumether/Essigester 7:3).

Beispiel XI

2-Tetrazolyl-N-trifluoracetyl-pyrrolidin

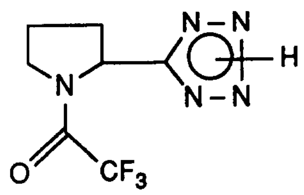

31,35 g = 32,6 ml (0,26 mol) Diethylaluminiumchlorid werden in 65 ml Toluol unter Schutzgas vorgelegt. Bei Raumtemperatur gibt man 29,95 g = 34,04 ml (0,26 mol) Trimethylsilylazid zu, und es wird 10 min bei Raumtemperatur nachgerührt. Bei 0 °C gibt man 25 g (0,13 mol) des Produkts aus Beispiel X, gelöst in 65 ml Toluol, hinzu. Die Reaktionsmischung wird 30 min bei 0 °C, 120 min bei Raumtemperatur und 60 min bei 40 °C gerührt. Der abgekühlte Ansatz wird so lange mit gesättigter Kaliumfluorid-Lösung versetzt, bis keine Gasentwicklung mehr zu erkennen ist.

Die Reaktionsmischung wird in 600 ml $H_2O$ gegeben und bis pH 4 angesäuert und 3 x mit 100 ml Essigester extrahiert. Die vereinigten organischen Phasen werden mit 50 ml n-Hexan versetzt. Um die Azide zu entfernen, wird ca 1/3 des Lösungsmittels über eine Destillationsbrücke ohne Kühlung abdestilliert. Der Rückstand wird über Magnesiumsulfat getrocknet und am Rotationsverdampfer vom Lösungsmittel befreit.
Man erhält 18,54 g der Titelverbindung (60,6 % der Theorie).
$R_f$: 0,4 (Toluol/Essigester 1:1).

Beispiel XII

N-Trifluoracetyl-2-[N-trityl-tetrazolyl]pyrrolidin

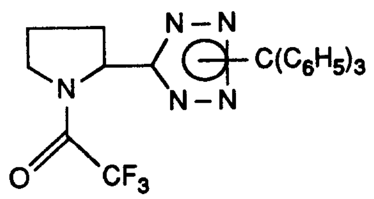

16,23 g (0,069 mol) des Produktes aus Beispiel XI und 10,47 g = 14,35 ml (0,1035 mol) Triethylamin werden in 70 ml Methylenchlorid vorgelegt. Dann gibt man 19,83 g (0,069 mol) Triphenylmethylchlorid zu. Man läßt die Reaktionsmischung 1,5 h bei Raumtemperatur nachrühren, verdünnt mit Methylenchlorid und extrahiert mit pH = 5 Pufferlösung (3 x 50 ml). Die organische Phase wird über Magnesiumsulfat getrocknet. Das Lösungsmittel wird am Rotationsverdampfer abgezogen. Der Rückstand wird mit Ether verrührt. Die entstandenen Kristalle werden abgesaugt und getrocknet.
Man erhält 24,65 g der Titelverbindung (75 % der Theorie).
$R_f$: 0,53 (Petrolether/Essigester 7:3).

Beispiel XIII

2-(N-Trityl-tetrazolyl)pyrrolidin

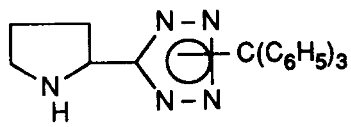

24 g (0,05 mol) des Produktes aus Beispiel XII werden unter Schutzgas in 100 ml Ethanol vorgelegt. Bei 0 ° C gibt man portionsweise 2,84 g (0,075 mol) Natriumborhydrid zu. Der Ansatz wird aufgetaut und 1 h bei Raumtemperatur gerührt. Man versetzt mit 6 µl Essigsäure und gibt die ganze Reaktionsmischung in 500 ml Pufferlösung pH 9. Der Ansatz wird mit 3 x 75 ml Methylenchlorid extrahiert. Die vereinigten organischen Phasen werden über Magnesiumsulfat getrocknet und am Rotationsverdampfer vom Lösungsmittel befreit. Der Rückstand wird an Kieselgel 60 F254 chromatographiert. Petrolether/Essigester (7:3). Die entsprechenden Fraktionen werden eingeengt und getrocknet.
Man erhält 7,16 g der Titelverbindung (37,5 % der Theorie).
$R_f$: 0,22 (Essigester).

Beispiel XIV

4-Brommethyl-3-chlor-benzolsulfonsäure-2-[trityl-tetrazolyl]pyrrolidinid

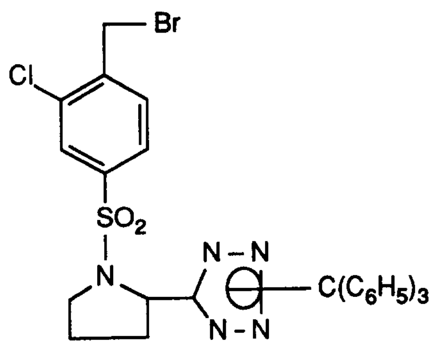

In Analogie zur Vorschrift des Beispiels III erhält man aus 3,19 g (10,5 mmol) der Verbindung aus Beispiel II und 4 g (10,5 mmol) der Verbindung aus Beispiel XIII 6,49 g der Titelverbindung (95 % der Theorie).
$R_f$: 0,53 (Petrolether/Essigester 7:3).

Beispiel XV

4-(Brommethyl)-3-fluorbenzolsulfochlorid

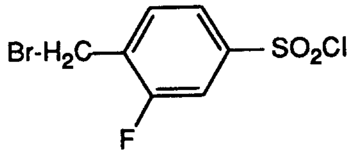

Man nimmt 20,9 g (0,1 mol) 3-Fluor-4-methylbenzolsulfochlorid in 200 ml Tetrachlorkohlenstoff auf und erhitzt nach Zugabe von 19,6 g (0,11 mol) N-Bromsuccinimid und 0,3 g Dibenzoylperoxid für 5 h unter Rückfluß. Nach dem Abkühlen werden die Feststoffe abfiltriert und das Filtrat vom Lösemittel befreit.

Flashchromatographie Petrolether/Toluol (4:1), 50 µm Korngröße, ergibt 12,4 g (44 % der Theorie) der Titelverbindung.
$R_f$: 0,42 (Petrolether/Toluol 3:1).

Beispiel XVI

4-(Brommethyl)-3-trifluormethylbenzolsulfochlorid

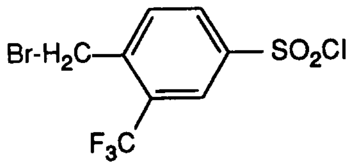

Man nimmt 64,6 g (0,25 mol) 3-Trifluormethyl-4-methylbenzolsulfochlorid in 500 ml Tetrachlorkohlenstoff auf und erhitzt nach Zugabe von 44,5 g (0,25 mol) N-Bromsuccinimid und 0,4 g ABN für 24 h unter Rückfluß. Nach dem Abkühlen werden die Feststoffe abfiltriert und das Filtrat vom Lösemittel befreit. Flashchromatographie Petrolether/Toluol (4:1), 50 µm Korngröße, ergibt 33,9 g (40 % der Theorie) der Titelverbindung.
$R_f$: 0,41 (Petrolether/Toluol 3:1).

Beispiel XVII

(S)-4-(Brommethyl)-3-fluorbenzolsulfonyl-N-2-(tert.butoxy-carbonyl)pyrrolidinid

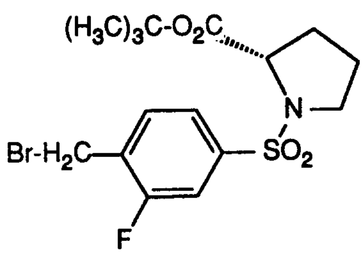

In Analogie zur Vorschrift aus Beispiel III erhält man aus 8,6 g (30 mmol) der Verbindung aus Beispiel XV und 5,1 g (30 mmol) S-Prolin-tert.-butylester 12,7 g (100 % der Theorie) der Titelverbindung.
$R_f$: 0,57 (Petrolether/Essigester 7:3).

Beispiel XVIII

(S)-4-(Brommethyl)-3-trifluormethylbenzolsulfonyl-N-2-(tert.-butoxycarbonyl)pyrrolidinid

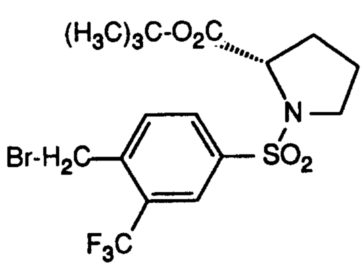

In Analogie zur Vorschrift des Beispiels III erhält man aus 16,9 g (50 mmol) der Verbindung aus Beispiel XVI und 8,6 g (50 mmol) S-Prolin-tert.-butylester 23,6 g (100 % der Theorie) der Titelverbindung.
$R_f$: 0,63 (Petrolether/Essigester 7:3).

Beispiel XIX

(S)-4-Carboxy-3-hydroxybenzolsulfonyl-N-2-(tert.-butoxycarbonyl)pyrrolidinid

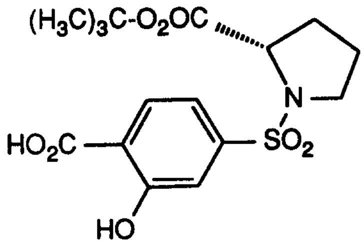

In Analogie zur Vorschrift des Beispiels III erhält man aus 23,7 g 4-Carboxy-3-hydroxybenzolsulfochlorid (100 mmol) und 17,1 g (100 mmol) S-Prolin-tert.-butylester 30,0 g (81 % der Theorie) der Titelverbindung.
$R_f$: 0,18 (Aceton)

Beispiel XX

(S)-4-Benzoyloxycarbonyl-3-benzyloxybenzolsulfonsäure-N-2-(tert.-butoxycarbonyl)pyrrolidinid

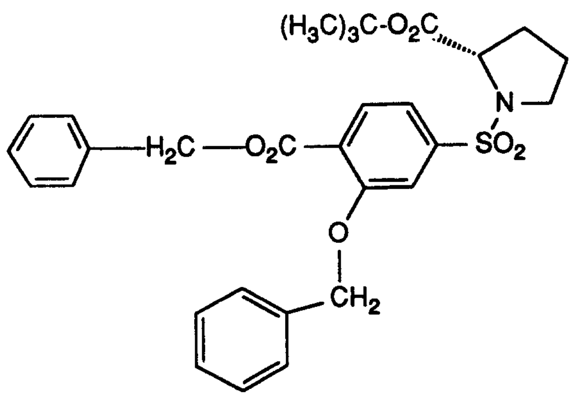

25,3 g (68 mmol) der Verbindung aus Beispiel XIX gelöst in 200 ml DMF werden mit 28,3 g $K_2CO_3$ (204 mmol) und 25,7 g (150 mmol) Benzylbromid versetzt. Man läßt die Reaktionsmischung 2 Stunden bei 75 °C nachrühren, gibt nach dem Abkühlen 1 l Wasser hinzu, extrahiert mit Essigester (3 x 400 ml), wäscht mit Wasser (5 x 400 ml), trocknet über $MgSO_4$, filtriert und zieht alles Flüchtige im Vakuum ab. Man reinigt durch Flash-Chromatographie (Petrolether/$CH_2Cl_2$ 5:1 und Petrolether/Essigester 6:1, 50 µm Teilchengröße). Zur weiteren Reinigung wird das Produkt aus 600 ml eines Lösungsmittelgemisches (Petrolether/Essigester 6:1) umkristallisiert und man erhält 35,5 g (95 % der Theorie) der Titelverbindung.
$R_f$ = 0,53 (Petrolether/Essigester 7:3).

Beispiel XXI

(S)-4-(Hydroxymethyl)-3-benzyloxybenzolsulfonsäure-N-2-(tert.-butoxycarbonyl)pyrrolidinid

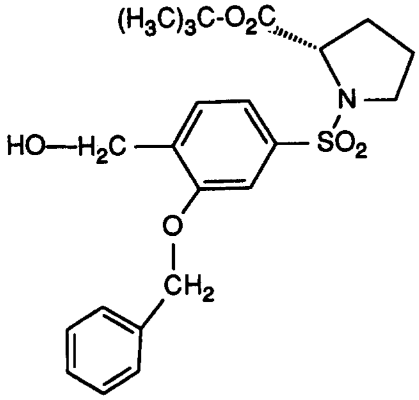

11,03 g (20 mmol) der Verbindung aus Beispiel XX werden in 100 ml Diglycene gelöst und nach Zugabe von 1,51 g (40 mmol) Natriumborhydrid und 1,68 g (40 mmol) LiCl für 4 Stunden bei 70 °C nachgerührt. Nach dem Abkühlen wird das Reaktionsgemisch mit 500 ml Wasser versetzt und mit 1 N HCl bis pH 3 angesäuert. Es wird mit Ether extrahiert (3 x 300 ml), mit Wasser gewaschen (6 x 300 ml), über $MgSO_4$ getrocknet und das Filtrat vom Lösemittel befreit. Der Rückstand wird an Kieselgel 60 F 254 chromatographiert. Petrolether/Essigester (7:3). Die entsprechenden Fraktionen werden eingeengt und getrocknet. Man erhält 5,0 g (56 % der Theorie) der Titelverbindung.
$R_f$: 0,36 (Petrolether/Essigester 7:3).

Beispiel XXII

(S)-4-(Brommethyl)-3-benzyloxybenzolsulfonsäure-N-2-(tert.-butoxycarbonyl)pyrrolidinid

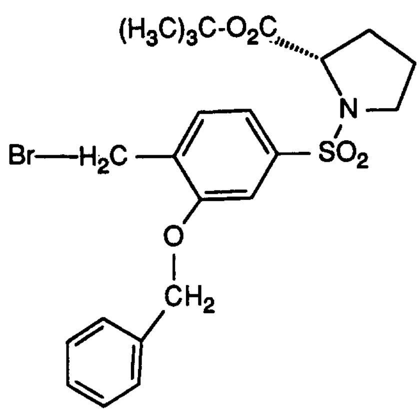

2,24 g (5 mmol) der Verbindung aus Beispiel XXI werden unter Schutzgas in 20 ml absolutem DMF vorgelegt. Bei 0 °C gibt man 2,53 g (6 mmol) Triphenylphosphindibromid hinzu. Das Reaktionsgemisch wird 1 Stunde bei Raumtemperatur gerührt. Man versetzt mit 200 ml Wasser, extrahiert mit Essigester (3 x 80 ml), wäscht mit Wasser (5 x 60 ml), trocknet über $MgSO_4$, filtriert und zieht alles Flüchtige im Vakuum ab. Man reinigt durch Flash-Chromatogaphie ($CH_2Cl_2$, 50 µm Teilchengröße) und erhält 2,55 g (100 % der Theorie) der Titelverbindung.
$R_f$: 0,56 (Petrolether/Essigester 7:3).

Herstellungsbeispiele

Beispiel 1

4-[(2-Butyl-4-chlor-5-formylimidazolyl)methyl]benzolsulfonyl-N-pyrrolidinid

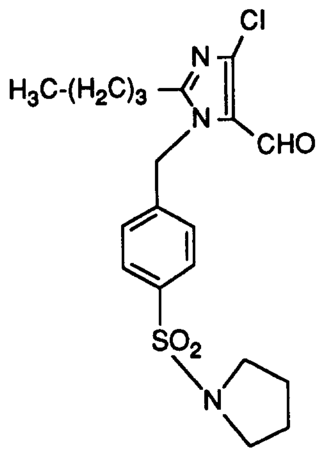

1,1 g (6,0 mmol) 2-Butyl-4-chlor-5-formylimidazol werden in 12 ml Dimethylformamid mit 180 mg (6,0 mmol) einer 80%igen Dispension von Natriumhydrid in Mineralöl versetzt und 30 min bei 20 ° C gerührt. Man kühlt auf 0 ° C und gibt 1,8 g (6,0 mmol) der Verbindung aus Beispiel III in 15 ml DMF zu. Man rührt 2,5 h bei 20 ° C nach und gießt die Reaktionsmischung auf Eis, extrahiert mit Essigester (3 x 50 ml) und wäscht die vereinigten organischen Phasen mit gesättigter Kochsalzlösung (5 x 50 ml), trocknet über $MgSO_4$, filtriert und zieht alles Flüchtige im Vakuum ab. Das Rohprodukt wird durch Flashchromatographie gereinigt (Petrolether / Essigester 10:1 -> 3:1,50 µ Teilchengröße), und man erhält 1,1 g (60% der Theorie) der Titelverbindung.
$R_f$ = 0,14 (Toluol)

Beispiel 2

4-[(2-Butyl-4-chlor-5-formylimidazolyl)methyl]benzosulfonyl-N-piperidinid

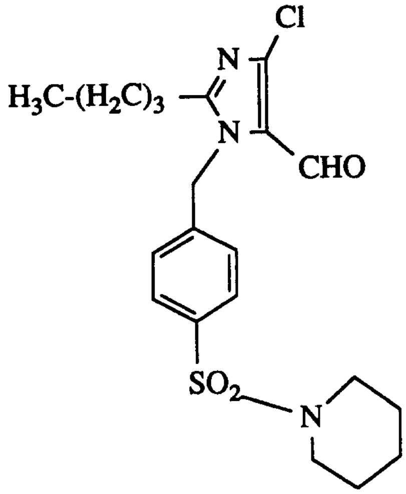

In Analogie zur Vorschrift des Beispiels 1 erhält man aus 3,8 g (12,0 mmol) der Verbindung aus Beispiel III und 2,2 g 2-Butyl-4-chlor-5-formylimidazol 3,1 g (61% der Theorie) der Titelverbindung.
$R_f$ = 0,39 (Petrolether / Essigester 7:3)

Beispiel 3

(S)-4-[(2-Butyl-4-chlor-5-formylimidazolyl)methyl]benzolsulfonyl-N-(2-tert.-butoxycarbonyl)pyrrolidinid

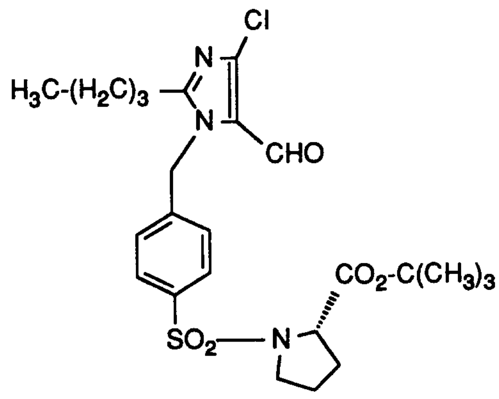

In Analogie zur Vorschrift des Beispiels 1 erhält man aus 9,1 g (23 mmol) der Verbindung des Beispiels V und 3,0 g (16 mmol) 2-Butyl-4-chlor-5-formylimidazol 6,0 g (74% der Theorie) der Titelverbindung.
$R_f$ = 0,61 (Petrolether / Essigester 7:3)

Beispiel 4

rac-4-[(2-Butyl-4-chlor-5-formylimidazolyl)methyl]benzolsulfonyl-N-(2-tert.-butoxycarbonyl)piperidinid

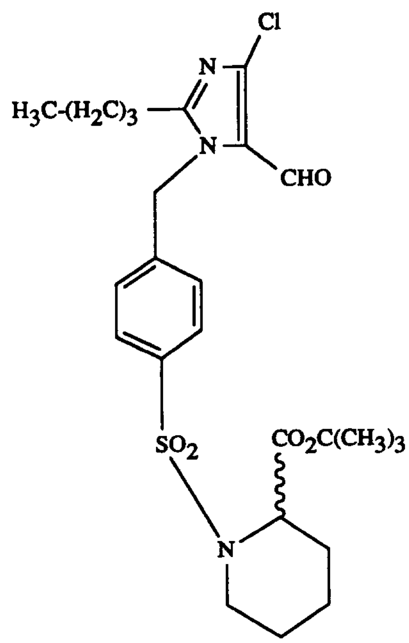

In Analogie zur Vorschrift des Beispiels 1 erhält man aus 7,4 g (18 mmol) der Verbindung aus Beispiel VI und 3,3 g (18 mmol) 2-Butyl-4-chlor-5-formylimidazol 4,9 g (53% der Theorie) der Titelverbindung.
$R_f$ = 0,08 (Petrolether / Essigester 7:1)

Beispiel 5

(S)-4-[(2-Butyl-4-chlor-5-formylimidazolyl)methyl]-3-chlorbenzolsulfonyl-N-(2-tert.butoxycarbonyl)pyrrolidinid

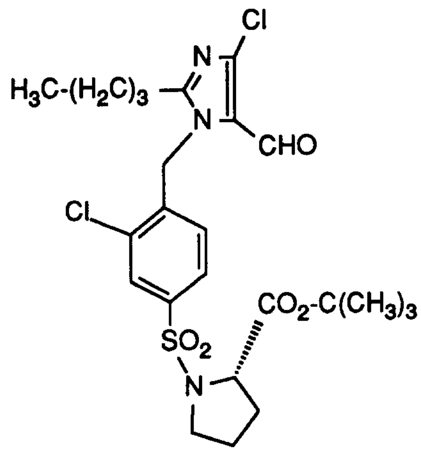

In Analogie zur Vorschrift des Beispiels 1 erhält man aus 6,6 g (15 mmol) der Verbindung aus Beispiel VII und 2,2 g (12 mmol) 2-Butyl-4-chlor-5-formylimidazol 2,7 g (42% der Theorie) der Titelverbindung.
$R_f$ = 0,75 (Dichlormethan / Essigester 10:1)

Beispiel 6

rac-4-[(2-Butyl-4-chlor-5-formylimidazolyl)methyl]-3-chlorbenzolsulfonyl-N-(2-tert.butoxycarbonyl)piperidinid

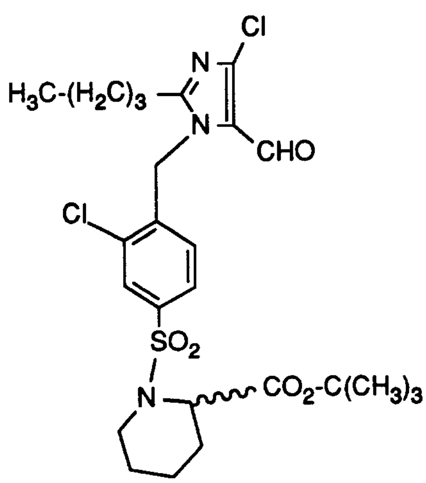

In Analogie zur Vorschrift des Beispiels 1 erhält man aus 6,8 g (15 mmol) der Verbindung aus Beispiel VIII und 2,2 g (12 mmol) 2-Butyl-4-chlor-5-formylimidazol 2,4g (26% der Theorie) der Titelverbindung. $R_f$ = 0,87 (Dichlormethan / Essigester 10:1)

Beispiel 7

rac-4-[5-Carboxy-4-chlor-2-butylimidazolyl)methyl]benzolsulfonyl-N-(2-tert.butoxycarbonyl)piperidinid

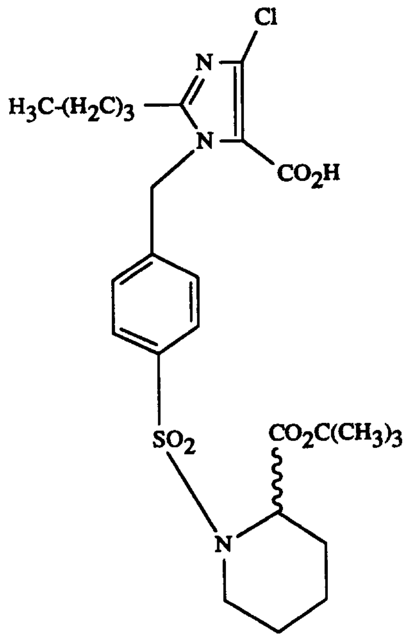

255 mg (0,5 mmol) der Verbindung aus Beispiel 3 werden in 3 ml tert.Butanol gelöst und mit 2 ml einer 1,25 M $NaH_2PO_4$-Lösung (eingestellt auf pH = 7 mit NaOH) versetzt, gefolgt von der Zugabe von 3 ml einer 1 M $KMnO_4$-Lösung. Man rührt 10 min bei 20 °C nach und versetzt mit 5 ml einer gesättigten $Na_2SO_4$-Lösung. Man stellt mit konz. HCl den pH-Wert auf 4 ein und extrahiert mit Essigester (3 mal 50 ml). Die vereinigten organischen Phasen werden mit $H_2O$ gewaschen (3 x 50 ml), über $MgSO_4$ getrocknet und vom Lösemittel befreit. Man erhält 258 mg (98% der Theorie) der obigen Titelverbindung.
$R_f$ = 0,14 (Dichlormethan / MeOH 10:1)

Beispiel 8

rac-4-[5-Carbonyl-4-chlor-2-butylimidazolyl]methylbenzolsulfonyl-N-(2-carboxy)piperidid

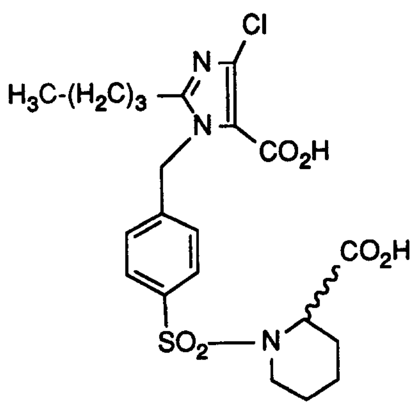

158 mg (0,3 mmol) der Verbindung aus Beispiel 7 werden in 10 ml Dichlormethan gelöst und mit 2 ml Trifluoressigsäure versetzt. Man rührt 4 h bei 20 °C nach, versetzt mit 50 ml Dichlormethan und wäscht mit Eiswasser (3 x 50 ml), trocknet über $MgSO_4$, filtriert, zieht alles Flüchtige im Vakuum ab und erhält 101 mg (72% der Theorie) der Titelverbindung.
$R_f$ = 0,10 ($CH_2Cl_2$/MeOH 3:1)

Beispiel 9

rac-4-[2-Butyl-4-chlor-5-(hydroxymethyl)imidazolyl]methyl-3-chlorbenzolsulfonyl-N-(2-tert.butoxycarbonyl)-piperidid

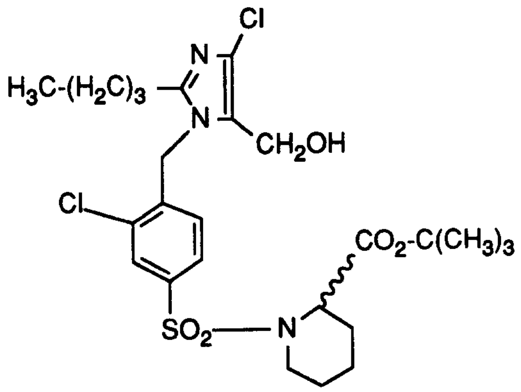

223 mg (0,4 mmol) der Verbindung aus Beispiel 6 werden in 5 ml THF gelöst und mit 30 mg (0,8 mmol) $NaBH_4$ in 2 ml 0,01 N NaOH versetzt. Man rührt 30 min bei 20 °C nach und versetzt anschließend unter Eiskühlung tropfenweise mit 1 N HCl bis zum Ende der Gasentwicklung. Die organische Phase wird abgetrennt und die wäßrige Phase mit Essigester (2 x 50 ml) extrahiert. Die vereinigten organischen Phasen werden mit $H_2O$ gewaschen (3 x 50 ml), über $MgSO_4$ getrocknet, filtriert und vom Lösemittel befreit. Man erhält 214 mg (96% der Theorie) der Titelverbindung.

$R_f$ = 0,35 ($CH_2Cl_2$/Essigester 5:1)

In Analogie zu den Vorschriften der Beispiele 1 - 9 werden die in Tabelle 1 aufgeführten Verbindungen hergestellt:

Tabelle 1:

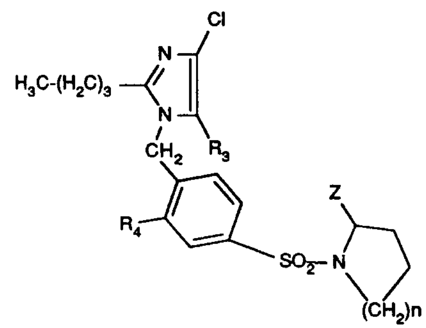

| Bsp.-Nr. | $R^3$ | $R^4$ | n | Z | Konfig. | Ausbeute** (% d.Th.) | $R_f$ *LM |
|---|---|---|---|---|---|---|---|
| 10 | $-CH_2OH$ | H | 1 | H | - | 79,4 | 0,18 [a] |
| 11 | $-CH_2OH$ | H | 2 | H | - | 82,2 | 0,14 [a] |
| 12 | $-CH_2OH$ | Cl | 1 | $-CO_2C(CH_3)_3$ | S | 96,3 | 0,28 [b] |
| 13 | $-CH_2OH$ | Cl | 2 | $-CO_2C(CH_3)_3$ | rac | 95,5 | 0,32 [b] |
| 14 | $-CH_2OH$ | H | 1 | $-CO_2C(CH_3)_3$ | S | 88,3 | 0,16 [b] |
| 15 | $-CO_2H$ | H | 1 | $-CO_2H$ | S | 70,2 (2 Stufen) | 0,06 [c] |
| 16 | $-CO_2H$ | Cl | 2 | $-CO_2H$ | rac | 76,8 (2 Stufen) | 0,19 [c] |
| 17 | $-CO_2H$ | H | 1 | $-CO_2C(CH_3)_3$ | S | 98,0 | 0,13 [d] |
| 18 | $-CO_2H$ | Cl | 2 | $-CO_2C(CH_3)_3$ | rac | 86,3 | 0,22 [d] |

** ausgehend von Aldehyd (incl. Verseifung)

<u>Tabelle 1:</u> (Fortsetzung)

| Bsp.-Nr. | $R^3$ | $R^4$ | n | Z | Konfig. | Ausbeute (% d.Th.) | $R_f$* |
|---|---|---|---|---|---|---|---|
| 19 | $-CH_2OH$ | Cl | 1 | $-CO_2H$ | S | 97,9 | $0,11^d$ |
| 20 | $-CH_2OH$ | Cl | 2 | $-CO_2H$ | rac | 91,5 | $0,21^d$ |
| 21 | $-CH_2OH$ | H | 1 | $-CO_2H$ | S | 87,6 | $0,09^d$ |
| 22 | $-CO_2H$ | Cl | 1 | $-CO_2C(CH_3)_3$ | S | 77,0 | $0,17^d$ |
| 23 | $-CO_2H$ | H | 2 | $-CO_2C(CH_3)_3$ | rac | 85,4 | $0,19^d$ |
| 24 | $-CO_2H$ | Cl | 1 | $-CO_2H$ | S | 86,1 | $0,17^c$ |
| 25 | $-CO_2H$ | H | 2 | $-CO_2H$ | rac | 97,4 | $0,26^c$ |

Beispiel 26

(S)-4-[(2-Butyl-5-formylimidazolyl)methyl]-3-chlorbenzolsulfonyl-N-(2-tert.-butoxycarbonyl)pyrrolidinid

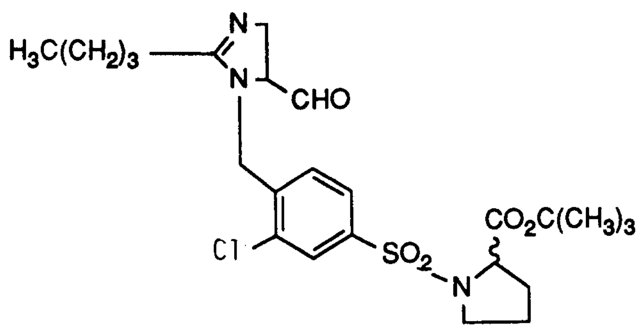

4,98 g (9,15 mmol) der Verbindung aus Beispiel 5 werden in 100 ml THF/50 ml Methanol gelöst und in Gegenwart von 1,24 g (9,15 mmol) Natriumacetat-Trihydrat und 0,5 g Palladium auf Aktivkohle (5 %ig) 1 h bei ca 3 bar Wasserstoffdruck hydriert. Danach wird vom Katalysator abfiltriert, eingeengt und der Rückstand über Kieselgel mit Essigester/Petrolether (1:1 und 2:1) gereinigt.
Ausbeute: 3,3 g (71 % der Theorie).
$R_f$ = 0,18 (Essigester/Petrolether = 1:1).

## Patentansprüche

**1.** Sulfonylbenzyl-substituierte Imidazole der allgemeinen Formel

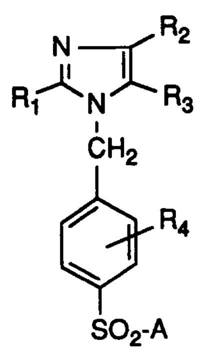

(I),

in welcher

$R^1$ für geradkettiges oder verzweigtes Alkyl oder Alkenyl mit jeweils bis zu 8 Kohlenstoffatomen steht, die gegebenenfalls durch Cycloalkyl mit 3 bis 6 Kohlenstoffatomen substituiert sind, oder
für Cycloalkyl mit 3 bis 8 Kohlenstoffatomen steht,

$R^2$ für Wasserstoff, Halogen oder für geradkettiges oder verzweigtes Perfluoralkyl mit bis zu 8 Kohlenstoffatomen steht,

$R^3$ für geradkettiges oder verzweigtes Alkyl mit bis zu 6 Kohlenstoffatomen steht, das durch Hydroxy oder geradkettiges oder verzweigtes Alkoxy mit bis zu 4 Kohlenstoffatomen substituiert ist, oder
für eine Gruppe der Formel $-CO-R^5$ oder $-CO-NR^6R^7$ steht,

worin

$R^5$ Wasserstoff, geradkettiges oder verzweigtes Alkoxy mit bis zu 8 Kohlenstoffatomen, Hydroxy, Benzyloxy oder Phenoxy bedeutet,

$R^6$ und $R^7$ gleich oder verschieden sind und Wasserstoff, geradkettiges oder verzweigtes Alkyl mit bis zu 8 Kohlenstoffatomen oder Phenyl bedeuten,

$R^4$ für Wasserstoff, Halogen oder geradkettiges oder verzweigtes Alkyl mit bis zu 8 Kohlenstoffatomen, oder
für geradkettiges oder verzweigtes Perfluoralkyl mit bis zu 6 Kohlenstoffatomen steht,
oder
für eine Gruppe der Formel -OX steht,
worin

X Wasserstoff, Benzyl, eine Hydroxyschutzgruppe oder geradkettiges oder verzweigtes Alkyl mit bis zu 8 Kohlenstoffatomen bedeutet,

A für einen über das Stickstoffatom gebundenen 3 bis 8-gliedrigen, gesättigten Heterocyclus mit bis zu 2 weiteren Heteroatomen aus der Reihe S, N oder O steht und der gegebenenfalls bis zu 2-fach gleich oder verschieden durch Perfluoralkyl mit bis zu 5 Kohlenstoffatomen oder durch einen Rest der Formel
$-SO_3H$,

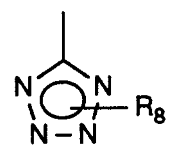 ,

$-CO-R^9$, $-CONR^{10}R^{11}$ oder

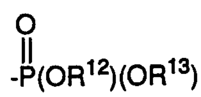

substituiert ist,
worin

$R^8$ Wasserstoff, geradkettiges oder verzweigtes Alkyl mit bis zu 6 Kohlenstoffatomen oder Triphenylmethyl bedeutet,

$R^9$ die oben angegebene Bedeutung von $R^5$ hat und mit dieser gleich oder verschieden ist,

$R^{10}$ und $R^{11}$ die oben angegebene Bedeutung von $R^6$ und $R^7$ haben und mit diesen gleich oder verschieden sind,
und

$R^{12}$ und $R^{13}$ gleich oder verschieden sind und Wasserstoff, geradkettiges oder verzweigtes Alkyl mit bis zu 8 Kohlenstoffatomen oder Phenyl bedeuten,

und deren Salze.

**2.** Sulfonylbenzyl-substituierte Imidazole nach Anspruch 1
wobei

$R^1$ für geradkettiges oder verzweigtes Alkyl oder Alkenyl mit jeweils bis zu 6 Kohlenstoffatomen steht, die gegebenenfalls durch Cyclopropyl, Cyclobutyl, Cyclopentyl oder Cyclohexyl substituiert sind, oder für Cyclopropyl, Cyclobutyl, Cyclopentyl oder Cyclohexyl steht,

$R^2$ für Wasserstoff, Fluor, Chlor, Brom oder für geradkettiges oder verzweigtes Perfluoralkyl mit bis zu 6 Kohlenstoffatomen steht,

$R^3$ für geradkettiges oder verzweigtes Alkyl mit bis zu 4 Kohlenstoffatomen steht, das durch Hydroxy, Methoxy oder Ethoxy substituiert ist, oder
für eine Gruppe der Formel $-CO-R^5$ oder $-CO-NR^6R^7$ steht,
worin

$R^5$ Wasserstoff, geradkettiges oder verzweigtes Alkoxy mit bis zu 6 Kohlenstoffatomen, Hydroxy, Benzyloxy oder Phenoxy bedeutet,

$R^6$ und $R^7$ gleich oder verschieden sind und Wasserstoff oder geradkettiges oder verzweigtes Alkyl mit bis zu 6 Kohlenstoffatomen bedeuten,

$R^4$ für Wasserstoff, Fluor, Chlor, Brom, geradkettiges oder verzweigtes Alkyl mit bis zu 6 Kohlenstoffatomen, oder für geradkettiges oder verzweigtes Perfluoralkyl mit bis zu 4 Kohlenstoffatomen steht, oder für eine Gruppe der Formel -OX steht, worin

X Wasserstoff, Benzyl, Acetyl oder geradkettiges oder verzweigtes Alkyl mit bis zu 6 Kohlenstoffatomen bedeutet,

A für über das Stickstoffatom gebundenes Azetidinyl, Piperidyl, Pyrrolidinyl oder Morpholinyl steht, die gegebenenfalls durch Trifluormethyl oder durch einen Rest der Formel

$-SO_3H$,

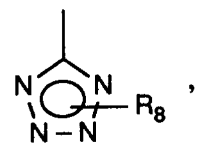

$-CO-R^9$, $-CONR^{10}R^{11}$ oder

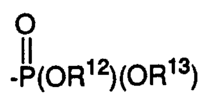

substituiert sind, worin

$R^8$ Wasserstoff, geradkettiges oder verzweigtes Alkyl mit bis zu 4 Kohlenstoffatomen oder Triphenylmethyl bedeutet,

$R^9$ die oben angegebene Bedeutung von $R^5$ hat und mit diesem gleich oder verschieden ist,

$R^{10}$ und $R^{11}$ die oben angegebene Bedeutung von $R^6$ und $R^7$ haben und mit diesen gleich oder verschieden sind, und

$R^{12}$ und $R^{13}$ gleich oder verschieden sind und Wasserstoff, geradkettiges oder verzweigtes Alkyl mit bis zu 6 Kohlenstoffatomen oder Phenyl bedeuten,

und deren Salze.

**3.** Sulfonylbenzyl-substituierte Imidazole nach Anspruch 1, wobei

$R^1$ für geradkettiges oder verzweigtes Alkyl oder Alkenyl mit jeweils bis zu 4 Kohlenstoffatomen oder Cyclopropyl steht,

$R^2$ für Wasserstoff, Fluor, Chlor oder für geradkettiges oder verzweigtes Perfluoralkyl mit bis zu 4 Kohlenstoffatomen steht,

$R^3$ für die $-CH_2OH$-Gruppe steht, oder für eine Gruppe der Formel $-CO-R^5$ oder $-CO-NR^6R^7$ steht, worin

$R^5$ Wasserstoff, geradkettiges oder verzweigtes Alkoxy mit bis zu 4 Kohlenstoffatomen, Hydroxy, Benzyloxy oder Phenoxy bedeutet,

$R^6$ und $R^7$ gleich oder verschieden sind und Wasserstoff oder geradkettiges oder verzweigtes Alkyl mit bis zu 4 Kohlenstoffatomen bedeuten,

$R^4$ für Wasserstoff, Fluor, Chlor, geradkettiges oder verzweigtes Alkyl mit bis zu 4 Kohlenstoffatomen, oder für geradkettiges oder verzweigtes Perfluoralkyl mit bis zu 3 Kohlenstoffatomen steht, oder

für eine Gruppe der Formel -OX steht,
worin

X Wasserstoff, Benzyl, Acetyl oder geradkettiges oder verzweigtes Alkyl mit bis zu 6 Kohlenstoffatomen bedeutet,

A für über das Stickstoffatom gebundenes Piperidyl, Pyrrolidinyl steht, die gegebenenfalls durch Trifluormethyl oder durch einen Rest der Formel
$-SO_3H$,

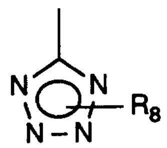

,

$-CO-R^9$, $-CONR^{10}R^{11}$ oder

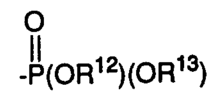

substituiert sind,
worin

$R^8$ Wasserstoff, Methyl, Ethyl oder Triphenyl bedeutet,

$R^9$ die oben angegebene Bedeutung von $R^5$ hat und mit diesem gleich oder verschieden ist,

$R^{10}$ und $R^{11}$ die oben angegebene Bedeutung von $R^6$ und $R^7$ haben und mit diesen gleich oder verschieden sind,
und

$R^{12}$ und $R^{13}$ gleich oder verschieden sind und Wasserstoff, geradkettiges oder verzweigtes Alkyl mit bis zu 4 Kohlenstoffatomen oder Phenyl bedeuten,

und deren Salze.

**4.** Sulfonylbenzyl-substituierte Imidazole nach Anspruch 1 zur therapeutischen Anwendung.

**5.** Verfahren zur Herstellung von Sulfonylbenzyl-substituierten Imidazolen nach Anspruch 1, dadurch gekennzeichnet, daß man Aldehyde der Formel (II)

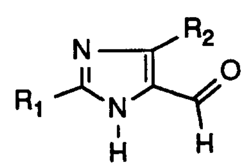

(II),

in welcher
$R^1$ und $R^2$ die in Anspruch 1 angegebene Bedeutung haben,
zunächst mit Verbindungen der allgemeinen Formel (III)

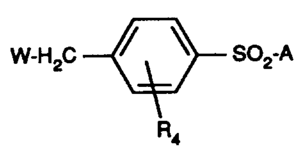

(III),

in welcher
$R^4$ und A die in Anspruch 1 angegebene Bedeutung haben

und

W für Halogen, vorzugsweise für Brom steht,

in inerten Lösemitteln, gegebenenfalls in Anwesenheit einer Base umsetzt,

und anschließend die Aldehydfunktion gegebenenfalls nach üblichen Methoden oxidiert oder reduziert,

im Fall der Ester, die Säuren nach üblicher Methode auch nachträglich verestert bzw. im Fall der Säuren, die Ester verseift,

im Fall der Amide, eine Amidierung, gegebenenfalls über eine aktivierte Carbonsäurestufe, in Anwesenheit einer Base und/oder eines Dehydratisierungsstoffes

und im Fall, daß $R^8 \neq H$ eine Alkylierung, anschließt.

**6.** Arzneimittel enthaltend mindestens ein Sulfonylbenzyl-substituiertes Imidazol nach Anspruch 1.

**7.** Arzneimittel nach Anspruch 6 zur Behandlung von Hypertonie und Atherosklerose.

**8.** Verfahren zur Herstellung von Arzneimitteln nach Anspruch 6, dadurch gekennzeichnet, daß man die Sulfonylbenzyl-substituierten Imidazole gegebenenfalls mit Hilfe von üblichen Hilfs- und Trägerstoffen in eine geeignete Applikationsform überführt.

**9.** Verwendung von Sulfonylbenzyl-substituierten Imidazolen nach Anspruch 1, zur Herstellung von Arzneimitteln.

**10.** Verwendung nach Anspruch 9, zur Herstellung von antihypertensiven und antiatherosklerotischen Arzneimitteln.